# EUROPÄISCHE PATENTSCHRIFT

(11) **EP 1 673 363 B1**
(45) Veröffentlichungstag und Bekanntmachung des Hinweises auf die Patenterteilung: **08.10.2008**
(21) Anmeldenummer: 04817145.8
(22) Anmeldetag: 13.10.2004
(51) Int. Cl.: C07D 311/94, C07D 315/00

(54) **VERFAHREN ZUR HERSTELLUNG VON 1-HYDROPEROXY-16-OXABICYCLO [10.4.0] HEXADECAN**
METHOD FOR PRODUCING 1-HYDROPEROXY-16-OXABICYCLO [10.4.0]HEXADECANE
PROCEDE DE PRODUCTION DE 1-HYDROPEROXY-16-OXABICYCLO [10.4.0]HEXADECANE

(30) Priorität: 16.10.2003 DE 10348168
(43) Veröffentlichungstag der Anmeldung: 28.06.2006
(73) Patentinhaber: Symrise GmbH & Co. KG, 37603 Holzminden (DE)
(72) Erfinder: LAMBRECHT, Stefan, 37619 Hehlen (DE); MARKS, Werner, 37647 Brevörde (DE); TOPP, Hans-Jürgen, 37603 Holzminden (DE); RICHTER, Norbert, Mount Pleasant, SC 29466 (US); KUHN, Walter, 37603 Holzminden (DE)
(74) Vertreter: Eisenführ, Speiser & Partner
(86) Internationale Anmeldenummer: PCT/EP2004/052521
(87) Internationale Veröffentlichungsnummer: WO 2005/035519

(56) Entgegenhaltungen:
- EP-A- 0 424 787
- EP-A- 1 375 491
- US-A- 3 856 815
- RÖMPP: "Chemie Lexicon, 10. Auflage" 1998, GEORG THIEME VERLAG , STUTTGART, DE , XP002318781 Seite 2831, Spalte 1

## Beschreibung

Die vorliegende Erfindung betrifft ein Verfahren zur Herstellung von 1-Hydroperoxy-16-oxabicyclo[10.4.0]hexadecan (DDP-Hydroperoxid, DDP-OOH) ausgehend von 13-Oxabicyclo[10.4.0]hexadec-1(12)-en (DDP), sowie ein Verfahren zur Herstellung von 11(12)-Pentadecen-15-olid aus dem so hergestellten DDP-OOH.

Die Verbindungen 11-Pentadecen-15-olid und 12-Pentadecen-15-olid sowie deren Gemische (11(12)-Pentadecen-15-olide) sind bekannte und wichtige Moschusriechstoffe. Dabei sind sowohl die jeweiligen (E)- als auch (Z)-Formen als auch deren Gemische geruchlich interessant. In EP-A 424 787 sind die geruchlichen Eigenschaften dieser Substanzen beschrieben. Es ist ebenfalls hinreichend bekannt, dass aus den 11(12)-Pentadecen-15-oliden mittels Hydrierung 15-Pentadecanolid (15-Hydroxypentadecansäurelacton) erhalten werden kann, welches ebenfalls als Moschusriechstoff verwendet wird.

Die Herstellung von 11(12)-Pentadecen-15-oliden läuft vorteilhaft ausgehend von 13-Oxabicyclo[10.4.0]hexadec-1(12)-en (DDP). Durch säurekatalysierter Addition von Wasserstoffperoxid an DDP wird 1-Hydroperoxy-16-oxabicyclo[10.4.0]hexadecan (DDP-Hydroperoxid, DDP-OOH) erhalten. Als zweiter Schritt in der Synthese zu den 11(12)-Pentadecen-15-oliden ist die Spaltung des DDP-OOH unter Bildung des makrocyclischen Rings zu sehen. Diese Spaltung wird meist in Gegenwart von Katalysatoren wie Cu(OAc)₂ und gegebenenfalls FeSO₄ durchgeführt. Wird diese Reaktionsstufe rein thermisch durchgeführt, so enthält das Reaktionsprodukt erhebliche Mengen der gesättigten Verbindung 15-Pentadecanolid, welche zwar ein Moschusriechstoff ist, jedoch andere geruchliche Eigenschaften als die 11(12)-Pentadecen-15-olide aufweist und daher nur in möglichst geringen Mengen entstehen sollte. Darüber hinaus ist bei der rein thermischen Spaltung eine hohe Rückstandsbildung (z.B. Destillationssumpf) nachteilig.

DDP wird üblicherweise unter Wasserabspaltung durch säurekatalysierte Cyclisierung von 2-(3-Hydroxypropyl)-1-cyclododecanon (OCP) erhalten, welches wiederum durch radikalische Addition von Allylalkohol an Cyclododecanon synthetisiert werden kann (z.B. in DE-OS 2136 496).

Der Prozess zur Herstellung der 11(12)-Pentadecen-15-olide kann durch das folgende Schema verdeutlicht werden:

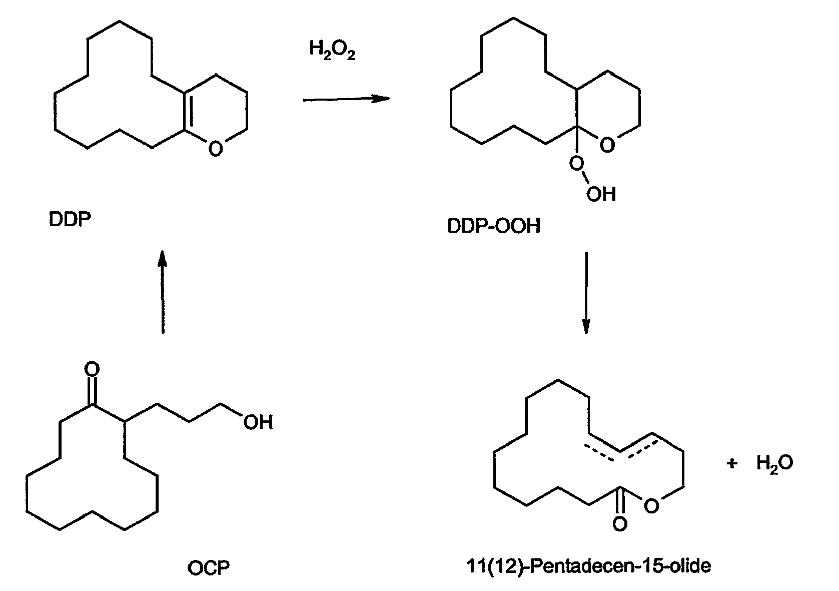

In EP-A 424 787 wurde OCP in 4,6 Gewichtequivalenten Eisessig bei Umgebungstemperatur homogenisiert, eine kalte 25 Gew. -%ige wässrige Lösung von Schwefelsäure (etwa 51 mol% (etwa 21 Gew.-%) bezogen auf OCP) zugegeben und die Reaktionsmischung anschließend auf 0°C abgekühlt Danach wurden 1,65 molare Equivalente an H₂O₂ (70 Gew.%ige Lösung) zugegeben, wobei die Temperatur auf 7°C anstieg. Nach einer kurzen Nachreaktionszeit wurde der gebildete Feststoff (DDP-OOH) abfiltriert, dieser mit Wasser und wässriger NaHCO₃-Lösung gewaschen und getrocknet; die Ausbeute betrug 80 %.

Die Spaltung des DDP-OOH erfolgte derart, dass das DDP-OOH in eine gesättigte Lösung von Cu(OAc)₂ in Methanol (hergestellt aus etwa 94 mol% Cu(OAc)₂ und 12,3 Gewichtsteilen Methanol bezogen auf das DDP-OOH; die Konzentration an DDP-OOH in dieser Methanol-Menge lag bei etwa 0,25 mol/l), portionsweise eingebracht wurde. Es folgte die Zugabe von 2 Portionen FeSO₄ (je knapp 20 mol% bezogen auf DDP-OOH) und Rühren bei Umgebungstemperatur über Nacht. Zur Aufarbeitung wurde auf gesättigte wässrige NaCl-Lösung gegeben, mit Diisopropylether extrahiert und dieser Extrakt mit gesättigter wässriger NaHCO₃-Lösung und gesättigter wässriger NaCl-Lösung gewaschen. Nach Trocknung und fraktionierter Destillation wurden 73 % der Theorie an 11 (12)-Pentadecen-15-oliden erhalten, die noch 8% 15-Pentadecanolid enthielten.

In Russ. Chem. Bull. 1998, 47, 1166-1169 wurde DDP in 5,2 Gewichtequivalenten Eisessig bei 0°C vorgelegt und eine Mischung bestehend aus einer 50 Gew.%igen wässrigen Lösung von Schwefelsäure (etwa 26 mol% (= 11 Gew.-%) bezogen auf DDP) und 30 Gew.%igem Wasserstoffperoxid (etwa 1,89 molare Equivalente) zugegeben. Nach einer kurzen Nachreaktionszeit wurde der gebildete Feststoff (DDP-OOH) abfiltriert, dieser mit einer 50 %igen Essigsäurelösung (80 Gew.% bezogen auf DDP) und anschließend mehrfach mit Wasser (4 Waschvorgänge mit je 2 Gewichtsteilen Wasser bezogen auf DDP) bis zur Neutralität des Waschwassers gewaschen. Nach Trocknung des Feststoffes wurden 85 % der Theorie an DDP-OOH erhalten, das eine 96 %ige Reinheit aufwies.

Die Spaltung des DDP-OOH erfolgte derart, dass eine Suspension aus 1 Anteil DDP-OOH und etwa 3,8 Gewichtsanteilen 4-Methylpentan-2-on (MIBK) über einen längeren Zeitraum in eine siedende Lösung von Cu(OAc)₂ in etwa 3,8 Gewichtsanteilen MIBK (bezogen auf DDP-OOH) eindosiert wurde. Die Menge an Cu(OAc)₂ wurde im Bereich von 0,15 bis 7,0 mol%, bezogen auf das DDP-OOH, variiert, wobei bei 5 mol% Cu(OAc)₂ nach Angabe der Autoren das Optimum lag. Nach 3 Stunden der Nachreaktionszeit bei Siedehitze wurde das Reaktionsgemisch abgekühlt und von den ausgefallenen Kupfersalzen befreit. Das Filtrat wurde mit heißem Wasser (2 Waschvorgänge mit je 7,7 Gewichtsequivalenten Wasser bezogen auf DDP-OOH) gewaschen und eingeengt. Es wurde unter Verwendung von 5 mol% Cu(OAc)₂ eine Rohausbeute an 11(12)-Pentadecen-15-oliden von 96,5 % der Theorie erhalten.

Nachteile dieser Verfahren sind unter den Reaktionsbedingungen der Spaltung von DDP-OOH insbesondere das Ausfallen elementaren Kupfers und/oder unlöslicher Kupferverbindungen sowie die großen Mengen der in den Reaktionen verwendeten Reagenzien und Hilfsstoffe. Als weitere Nachteile sind beispielsweise die vielen, teils aufwendigen, Prozessschritte zu nennen und die unbefriedigende Raum-Zeit-Ausbeute. Das Waschen der DDP-OOH Kristalle, teils bis zur Neutralität des Waschwassers ist nicht nur aufwendig und umweltbelastend, sondern macht auch deutlich, dass Säurereste im DDP-OOH für die Folgestufe der Fragmentierung zu vermeiden sind. Insbesondere nachteilig ist die Isolierung des DDP-Hydroperoxids, die nicht nur aufwendig, sondern auch sicherheitstechnisch problematisch ist, da Hydroperoxide eine hohes Gefährdungspotential aufweisen.

Die bekannten Syntheseverfahren sind daher für eine industrielle Umsetzung ungeeignet. Ein technisches Verfahren, das auf einfache und kostengünstige Weise 11(12)-Pentadecen-15-olide liefert, ist deshalb von großem wirtschaftlichem Interesse.

Mit der vorliegenden Erfindung gelingt es, die genannten Nachteile zu überwinden, und ein technisch günstiges Verfahren bereitzustellen. Das erfindungsgemäße Verfahren ist besonders für den Einsatz im industriellen Maßstab geeignet.

Gegenstand der vorliegenden Erfindung ist ein Verfahren zur Herstellung von 1-Hydroperoxy-16-oxabicyclo[10.4.0]hexadecan (DDP-OOH) wobei 13-Oxabicyclo[10.4.0]hexadec-1(12)-en (DDP) und Wasserstoffperoxid in einem Verdünnungsmittel in Gegenwart einer starken Säure umgesetzt werden, das Verdünnungsmittel einen pKa-Wert von größer oder gleich 4,5 und die starke Säure einen pKa-Wert von kleiner oder gleich 1,5 aufweist, wobei nach erfolgter Umsetzung, also typischerweise nach beendeter Zugabe des Wasserstoffperoxids und der sich gegebenenfalls anschließenden Nachreaktionszeit, die starke Säure mit mindestens 0,9 Molequivalenten einer Base neutralisiert wird.

Unter dem pKa-Wert wird der negative dekadische Logarithmus der Aciditätskonstante Ka in Wasser bei 25°C (298K) verstanden: pKa = -log₁₀ Ka. Der pKa-Wert ist ein Maß für die Stärke einer Säure.

Vorteilhaft sind starke Säuren, die einen pKa-Wert von kleiner oder gleich 1,0 aufweisen, bevorzugt sind hierbei Mineralsäuren und gerad- oder verzweigtkettige fluorierte Carbonsäuren.

Bevorzugte Mineralsäuren sind Salpetersäure, Salzsäure, Perchlorsäure und Schwefelsäure, besonders bevorzugt ist Schwefelsäure. Vorteilhaft sind hierbei wiederum 50 - 98 Gew.-%ige Schwefelsäuren.

Vorteilhafte fluorierte Carbonsäuren im Sinne der vorliegenden Erfindung sind gerad- oder verzweigtkettige Carbonsäuren, umfassen 2 bis 6 Kohlenstoffatome, enthalten mindestens ein Fluoratom und weisen einen pKa-Wert von kleiner oder gleich 1,5 auf. Bevorzugt sind hochfluorierte Carbonsäuren oder Perfluorsäuren mit einem pKa-Wert von kleiner oder gleich 1,0. Bei hochfluorierten Carbonsäuren sind mindestens 80%, bevorzugt mindestens 90%, der Wasserstoffatome der C-H-Bindungen durch Fluoratome ausgetauscht. Bevorzugte Perfluorsäuren sind Trifluoressigsäure, Pertluorpropionsäure, Perfluorbutansäure, Perfluorpentansäure, Perfluorhexansäure. Ganz besonders bevorzugt ist Trifluoressigsäure.

Daneben können natürlich auch andere starke Säuren, wie beispielsweise Trifluormethansulfonsäure, eingesetzt werden. Es können auch Mischungen von starken Säuren eingesetzt werden.

Die bevorzugte Menge an starker Säure, bezogen auf DDP, liegt im Bereich 0,1 bis 10 mol%, besonders bevorzugt im Bereich 0,5 bis 5 mol%.

Als Verdünnungsmittel für die Herstellung des DDP-OOH sind polar protische und polar aprotische Verdünnungsmittel besonders geeignet, vorteilhafterweise sind die Verdünnungsmittel bei 25°C flüssig. Bevorzugt sind polar protische Verdünnungsmittel.

Vorteilhafte Verdünnungsmittel weisen einen pKa-Wert im Bereich von größer oder gleich 4,5 und von kleiner oder gleich 26 auf. Bevorzugte Verdünnungsmittel weisen einen pKa-Wert im Bereich von größer oder gleich 4,5 und von kleiner oder gleich 18, besonders bevorzugt einen im Bereich von größer oder gleich 4,5 und von kleiner oder gleich 10 auf.

Es können auch Mischungen von erfindungsgemäßen Verdünnungsmitteln eingesetzt werden.

Bevorzugt sind organische Säuren, insbesondere gerad- oder verzweigtkettige organische Säuren mit 2 bis 6 Kohlenstoffatomen oder Gemische organischer Säuren mit 2 bis 6 Kohlenstoffatomen. Ganz besonders bevorzugte Verdünnungsmittel sind Essigsäure, Propionsäure oder ein Gemisch aus Essigsäure und Propionsäure.

Das erfindungsgemäß bevorzugte Gewichtsverhältnis von DDP zu Verdünnungsmittel liegt im Bereich 1:1 bis 1:8, bevorzugt bei 1:2 bis 1:4.

Neben dem Verdünnungsmittel enthält das Reaktionsmedium vorteilhafterweise zusätzlich Wasser. Das erfindungsgemäß bevorzugte Gewichtsverhältnis von DDP zu Wasser liegt im Bereich 10:1 bis 1:1, bevorzugt bei 5:1 bis 2:1.

Vor dem Einsatz des DDP-OOH in die Fragmentierungsstufe zu den 11 (12)-Pentadecen-15-oliden wird erfindungsgemäß die starke Säure mit mindestens 0,9 Molequivalenten einer Base neutralisiert, vorzugsweise vollständig. Es können zur Neutralisierung organische und anorganische Basen verwendet werden, die auch als Lösung eingesetzt werden können. Besonders bevorzugt sind Alkalihydroxide, Erdalkalihydroxide, Alkalicarbonate, Erdalkalicarbonate, Alkali- oder Erdalkalisalze einer gerad- oder verzweigtkettigen organischen Säure mit 1 bis 6 Kohlenstoffatomen. Besonders bevorzugt sind Natriumhydroxid, Kaliumhydroxid, Alkaliacetat, Alkalipropionat. Bevorzugte Alkali-Vertreter sind Natrium und Kalium.

Die bevorzugte Menge an Base, bezogen auf die starke Säure, liegt bei mindestens 1,0 Molequivalenten, besonders bevorzugt liegt die Menge im Bereich 1,0 bis 2,5 Molequivalenten; insbesondere im Bereich-1,005 bis 1,5 Molequivalenten. Es können auch Mischungen von Basen eingesetzt werden.

Für die Addition von H₂O₂ an DDP zu DDP-OOH kann Wasserstoffperoxid unterschiedlichen Gehalts verwendet werden. Typischerweise wird 10 bis 70 Gew.%iges wässriges Wasserstoffperoxid eingesetzt, bevorzugt ist 30 bis 55 Gew.%iges, bevorzugt sind 30 Gew.%iges und 50 Gew.%iges wässriges H₂O₂.

Die bevorzugte Menge an H₂O₂ beträgt 0,9 bis 2 Molequivalenten bezogen auf DDP und besonders bevorzugt 1,2 bis 1,6 Molequivalente.

Der Temperaturbereich, in dem die Addition von H₂O₂ durchgeführt wird, liegt vorzugsweise bei -20 bis +20°C, bevorzugt bei -10 bis +10°C. Der Temperaturbereich kann selbstverständlich vom Fachmann, je nach verwendetem Verdünnungsmittel, ausgewählt und optimiert werden. Eine Temperatur oberhalb von +20°C wird vorzugsweise vermieden, weil das gebildete DDP-OOH bei höheren Temperaturen weniger stabil ist und sich mit steigender Temperatur zunehmend zersetzt.

Als Ausgangsmaterial für die Herstellung von DDP-OOH kann auch - wie ähnlich gemäß EP-A 424 787 - OCP verwendet werden. Da bei der Cyclisierung zum DDP Wasser entsteht, ist es vorteilhaft, die Wassermenge entsprechend zu reduzieren.

Das nach dem erfindungsgemäßen Verfahren hergestellte DDP-OOH wird vorteilhafterweise ohne weitere Aufarbeitung oder Isolierung, d.h. in Form des rohen Reaktionsgemisches, in die Fragmentierungsstufe zu den 11(12)-Pentadecen-15-oliden eingebracht. Typischerweise fällt das rohe DDP-OOH Reaktionsgemisch in Form einer Suspension an. Dass die Verfahrensschritte der Isolierung und/oder des Neutralwaschens des DDP-OOH nicht notwendig sind, ist angesichts des Standes der Technik in besonderem Maße überraschend.

Ein weiterer Gegenstand der vorliegenden Erfindung ist ein Verfahren zur Herstellung von 11(12)-Pentadecen-15-oliden, bei dem ein Reaktionsgemisch, das nach dem zuvor beschriebenen erfindungsgemäßen Verfahren erhalten wurde, ohne Isolierung des DDP-OOH eingesetzt wird.

Vorteilhafterweise werden zur Fragmentierung des im Reaktionsgemisch enthaltenen DDP-OOH (Cu(I)- oder Cu(II)-Verbindungen eingesetzt.

Bei der Fragmentierung des DDP-OOH zu den 11(12)-Pentadecen-15-oliden eingesetzte Cu(I)-oder Cu(II)-Verbindungen sind dabei vorteilhafterweise solche, die unter den Reaktionsbedingungen der DDP-OOH-Umsetzung in dem verwendeten hochsiedendem Verdünnungsmittel in einem gewissen Ausmaß löslich sind. Solche Kupferverbindungen weisen bei 20°C in dem Verdünnungsmittel eine Löslichkeit von mindestens 0,5g /kg hochsiedendem Verdünnungsmittel auf, bevorzugt von mindestens 1g /kg. Die Kupferverbindungen können wasserfrei oder als Hydrate (Kristallwasser) eingesetzt werden. Die Wassermenge des Kristallwassers ist unkritisch.

Vorteilhaft sind Cu-(II)-verbindungen, bevorzugte sind solche mit organischen Resten. Neben Cu-(II)-2,4-pentandionat-Derivaten sind dabei Cu-(II)-carboxylate besonders geeignet. Bevorzugte Cu-(II)-2,4-pentandionate sind Cu-(II)-acetylacetonat, Cu-(II)-1,1,1-trifluoracetylacetonat und [Bis(2,2,6,6-tetramethyl-3,5-heptandionato)]-Cu-(II). Besonders bevorzugt sind Cu-(II)-carboxylate von Alkylcarbonsäuren mit 2 bis 5 Kohlenstoffatomen, insbesondere Cu-(II)-acetat und Cu-(II)-propionat.

Erfindungsgemäß können ein oder mehrere Kupferverbindungen verwendet werden. Die erfindungsgemäß vorteilhafte Menge der Kupferverbindungen bei der Umsetzung von DDP-OOH zu den 11(12)-Pentadecen-15-oliden beträgt 0,05 bis 4 mol% bezogen auf DDP-OOH, besonders vorteihaft 0,1 bis 2,5 mol%, bevorzugt 0,1 bis 1,5 mol% und besonders bevorzugt 0,3 bis 1,5 mol%, ganz besonders bevorzugt 0,5 bis 1,5 mol%.

Bevorzugte hochsiedende Verdünnungsmittel für die Umsetzung des DDP-OOH zu den 11(12)-Pentadecen-15-oliden sind polar und haben einen hohen Siedepunkt, so dass Sie bei der Fragmentierungsreaktion im Reaktor verbleiben. Der Siedepunkt des hochsiedenden Verdünnungsmittels liegt somit bevorzugt oberhalb des Siedpunktes des verwendeten Verdünnungsmittels der Reaktionsstufe zur Bildung des DDP-OOH, oberhalb des azeotropen Gemisches von Verdünnungsmittel und Wasser sowie oberhalb des Siedpunktes des Produktes. d.h. der 11(12)-Pentadecen-15-olide. Bevorzugt sind daher hochsiedende Verdünnungsmittel mit einem Siedepunkt von größer oder gleich 170°C bei einem Druck von 5 mbar.

Es wurde zudem gefunden, dass bestimmte hochsiedende Verdünnungsmittel ein Ausfallen von Kupfer und/oder unlöslichen Kupferverbindungen während der Spaltung unterdrücken. Bevorzugt wird insoweit die Umsetzung von DDP-OOH zu den 11(12)-Pentadecen-15-ofiden in Gegenwart von hochsiedenden Verdünnungsmitteln durchgeführt, die durch folgende Formel wiedergegeben werden können:

HX-[A]-YH

wobei
X und Yunabhängig voneinander O oder N-R bedeuten, wobei R = H oder ein organischer Rest mit 1 bis 10 Kohlenstoffatomen ist, und
A ein organischer Rest mit bis zu 100 Kohlenstoffatome ist.

Bevorzugt umfasst A bis zu 50 Kohlenstoffatome, besonders bevorzugt 10 bis 30 Kohlenstoffatome. Der Rest R umfasst bevorzugt 1 bis 4 Kohlenstoffatome, bevorzugt ist R = Methyl oder Ethyl. Bevorzugt sind auch hierbei hochsiedende Verdünnungsmittel mit einem Siedepunkt von größer oder gleich 170°C bei 5 mbar.

Der organische Rest A enthält bevorzugt die Heteroatome O oder N, bevorzugt in Form von Hydroxygruppen, Ethergruppen oder Aminogruppen, bevorzugt sind Ethergruppen und sekundäre Aminogruppen. An dem Kohlenstoffgerüst des Restes A können ein oder mehrere organische Gruppierungen angebracht sein, die unabhängig voneinander geradkettig, verzweigt, cyclisch, heterocyclisch, aromatisch oder heteroaromatisch sein können, wobei bei den heteroatomhaltigen Gruppierungen solche mit O oder N bevorzugt sind.

Vorteilhafte hochsiedende Verdünnungsmittel sind α,ω-Diole und α,ω-Aminoalkohole.

In einer besonders vorteilhaften Ausführungsform werden hochsiedende Verdünnungsmittel eingesetzt, die als Heteroatome ausschließlich Sauerstoff enthalten. Diese α,ω-Diole enthalten im Kohlenstoffgerüst des organischen Restes A bevorzugt mindestens 2 Sauerstoffatome, bevorzugt in Form von Ethergruppen.

Besonders bevorzugte hochsiedende Verdünnungsmittel sind Polyalkylenglykole, insbesondere Polyethylenglykole (PEG), Polypropylenglykole oder Polytetramethylenglykole (Polytetrahydrofurane), welche mindestens einen Siedepunkt von 170°C bei 5mbar aufweisen. Die Polyalkylenglykole sind bei höheren Molmassen polydispers und haben einen Molmassenbereich, z.B. weist PEG 1000 typischerweise einen Molmassenbereich von 950 bis 1050 auf. Ganz besonders bevorzugt sind Polyethylenglykole. In besonderem Maße bevorzugt sind PEG 400 bis PEG 1500, hierbei wiederum PEG 400, PEG 600, PEG 800 und PEG 1000. Diese Produkte sind handelsüblich verfügbar.

Erfindungsgemäß können ein oder mehrere hochsiedende Verdünnungsmittel verwendet werden. Die erfindungsgemäß vorteilhafte Menge an hochsiedendem Verdünnungsmittel bei der Umsetzung von DDP-OOH zu den 11(12)-Pentadecen-15-oliden beträgt 5 bis 100 Gew.% bezogen auf DDP-OOH, bevorzugt 10 bis 70 Gew.%, besonders bevorzugt 15 bis 60 Gew.% und ganz besonders bevorzugt 20 bis 50 Gew.-%.

Der Temperaturbereich, in dem die Spaltung durchgeführt wird, liegt vorteilhafterweise im Bereich von 70 bis 120°C. Bevorzugt wird die Spaltung bei Temperaturen im Bereich von 85 bis 110°C, besonders bevorzugt bei 90 bis 100°C durchgeführt.

Der vorteilhafte Druckbereich, in dem die Fragmentierung des DDP-OOH durchgeführt wird, liegt bei 0,01 mbar bis 2 bar. Bevorzugt wird das Verfahren bei Drücken unterhalb von 1013 mbar ausgeführt, insbesondere im Bereich von 50 bis 800 mbar.

Die Fragmentierung wird vorteilhaft durchgeführt, indem die DDP-OOH-Suspension zu einer Mischung aus der Kupferverbindung und dem hochsiedenden Verdünnungsmittel getropft wird.

Ein weiterer Vorteil des Verfahrens sind die geringen Abfallmengen. Abwasser fällt im Prozess nicht an, das bei der Herstellung von DDP-OOH verwendete Lösungsmittel kann nach Destillation wieder in den Prozess eingeschleust werden. Dies ist unter Umwelt- und Wirtschaftlichkeitsaspekten besonders vorteilhaft

Nach dem erfindungsgemäßen Verfahren können isolierte Ausbeuten an 11(12)-Pentadecen-15-olide von etwa 87 % d. Th. an 11(12)-Pentadecen-15-oliden (Summe der verschiedenen Isomere) erreicht werden. Zusätzlich werden typischerweise etwa 1-2 % Pentadecan-15-olid und 5-7 % DDP, das wieder in der Prozess eingeschleust werden kann, erhalten.

### Beispiele:

### Allgemeiner Versuchsaufbau:

Die Versuche wurden in Doppelmantelrührreaktoren durchgeführt, die über einen Kryostaten bzw. Thermostaten temperiert wurden. Der Bodenablass des Reaktors, in dem die Herstellung des Hydroperoxids stattfand, wurde über eine Schlauchpumpe mit einer Dosierleitung des Fragmentierungsreaktors verbunden.

### Beispiel 1:

633 g (2,85 mol) DDP, 1800 g Propionsäure, 180 g Wasser und 15 g Trifluoressigsäure werden im ersten Reaktor zusammengegeben und auf -10 °C abgekühlt. Bei -10 bis +10 °C werden 265 g einer 50 Gew.%igen wässrigen Lösung von Wasserstoffperoxid zugetropft. Es schließt eine Nachreaktionszeit von 1 Stunde an, in der das Hydroperoxid kristallisiert. Anschließend werden 53 g einer 10 Gew.%igen Natronlauge zugegeben.
Die entstandene Suspension wird innerhalb von 3 Stunden in den Fragmentierungs-Reaktor, in dem 200 g Polydiol 400 und 4 g Kupfer-(II)-Acetat-Monohydrat bei 90 °C vorgelegt wurden, gefördert. Ein Propionsäure/Wasser-Gemisch wird dabei abdestilliert.
Das auf diese Weise erhaltene Rohprodukt ergibt nach Destillation über eine 30 cm Füllkörperkolonne 665 g Destillat, dass 89 Gew.% 11(12)-Pentadecen-15-olide (Summe der verschiedenen Isomere, die Isomeren sind zu etwa 40%, 27% und 22% enthalten), 2 Gew.-% Pentandecan-15-olid und 5 Gew.-% DDP enthält. Dies entspricht einer Ausbeute von 87% der Theorie an 11(12)-Pentadecen-15-olid (Summe der Isomere).

### Beispiel 2:

633 g (2,85 mol) DDP, 1800 g Propionsäure, 180 g Wasser und 15 g Trifluoressigsäure werden im ersten Reaktor zusammengegeben und auf -10 °C abgekühlt. Bei -10 bis +10 °C werden 245 g einer 50 Gew.%igen wässrigen Lösung von Wasserstoffperoxid zugetropft. Es schließt eine Nachreaktionszeit von 1 Stunde an, in der das DDP-OOH kristallisiert. Anschließend werden 65 g einer 23 Gew.%igen Natriumpropionat-Lösung zugegeben.
Die entstandene Suspension wird innerhalb von 3 Stunden in den Fragmentierungs-Reaktor, in dem 200 g Polydiol 400 und 4 g Kupfer-(II)-Acetat-Monohydrat bei 90 °C vorgelegt wurden, gefördert. Ein Propionsäure/Wasser-Gemisch wird dabei abdestilliert.
Das auf diese Weise erhaltene Rohprodukt ergibt nach Destillation über eine 30 cm Füllkörperkolonne 662 g Destillat, dass 81 Gew.% 11(12)-Pentadecen-15-olid (Summe der verschiedenen Isomere), 1 Gew.% Pentandecan-15-olid und 7 Gew.% DDP enthält. Dies entspricht einer Ausbeute von 80 % d. Th. an 11(12)-Pentadecen-15-olid (Summe der verschiedenen Isomere, die Isomeren sind zu etwa 40%, 27% und 22% enthalten).

### Beispiel 3:

633 g (2,85 mol) DDP, 1800 g Propionsäure, 180 g Wasser und 6,6 g konzentrierter Schwefelsäure (96%ig) werden im ersten Reaktor zusammengegeben und auf -10°C abgekühlt. Bei -10 bis +10°C werden 245 g einer 50 Gew.-%igen wässrigen Lösung von Wasserstoffperoxid zugetropft. Es schließt eine Nachreaktionszeit von 1 Stunde an, in der das Hydroperoxid kristallisiert. Anschließend werden 63 g einer 10 Gew.-%igen Natronlauge zugegeben.
Die entstandene Suspension wird innerhalb von 3 Stunden in den Fragmentierungs-Reaktor, in dem 200 g Polydiol 400 und 4 g Kupfer-(II)-Acetat-Monohydrat bei 90 °C vorgelegt wurden, gefördert. Ein Propionsäure/Wasser-Gemisch wird dabei abdestilliert.

Das auf diese Weise erhaltene Rohprodukt ergibt nach Destillation über eine 30 cm Füllkörperkolonne 663 g Destillat, dass 88 Gew.-% 11(12)-Pentadecen-15-olid (Summe verschiedener Isomere), 1 Gew.% Pentandecan-15-olid und 3 Gew.-% DDP enthält. Dies entspricht einer Ausbeute von 86 % d. Th. an 11(12)-Pentadecen-15-olid (Summe der verschiedenen Isomere, die Isomeren sind zu etwa 40%, 27% und 22% enthalten).

## Patentansprüche

1. Verfahren zur Herstellung von 1-Hydroperoxy-16-oxabicyclo[10.4.0]hexadecan (DDP-OOH) wobei 13-Oxabicyclo[10.4.0]hexadec-1(12)-en (DDP) und Wasserstoffperoxid in einem Verdünnungsmittel in Gegenwart einer starken Säure umgesetzt werden, das Verdünnungsmittel einen pKa-Wert von größer oder gleich 4,5 und die starke Säure einen pKa-Wert von kleiner oder gleich 1,5 aufweist,
**dadurch gekennzeichnet, dass** nach erfolgter Umsetzung die starke Säure mit mindestens 0,9 Molequivalenten einer Base neutralisiert wird.

2. Verfahren nach Anspruch 1, **dadurch gekennzeichnet, dass** das Verdünnungsmittel einen pKa-Wert im Bereich von größer oder gleich 4,5 und von kleiner oder gleich 25 aufweist und vorzugsweise eine organische Säure ist.

3. Verfahren nach Anspruch 1, **dadurch gekennzeichnet, dass** das Verdünnungsmittel eine gerad- oder verzweigtkettige organische Säure mit 2 bis 6 Kohlenstoffatomen oder ein Gemisch organischer Säuren mit 2 bis 6 Kohlenstoffatomen ist.

4. Verfahren nach Anspruch 1, **dadurch gekennzeichnet, dass** das Verdünnungsmittel Essigsäure, Propionsäure oder ein Gemisch aus Essigsäure und Propionsäure ist.

5. Verfahren nach einem der Ansprüche 1 bis 4, **dadurch gekennzeichnet, dass** die starke Säure einen pKa-Wert von kleiner oder gleich 1,0 aufweist

6. Verfahren nach einem der Ansprüche 1 bis 5, **dadurch gekennzeichnet, dass** die starke Säure eine Mineralsäure oder eine gerad- oder verzweigtkettige fluorierte Carbonsäure, vorzugsweise eine perfluorierte Carbonsäure, ist.

7. Verfahren nach einem der Ansprüche 1 bis 6, **dadurch gekennzeichnet, dass** die starke Säure gewählt ist aus der Gruppe Schwefelsäure, Trifluoressigsäure, Perfluorpropionsäure, Pertluorbutansäure, Perfluorpentansäure, Perfluorhexansäure.

8. Verfahren nach einem der Ansprüche 1 bis 7, **dadurch gekennzeichnet, dass** eine wässrige Wasserstoffperoxid-Lösung mit einem Wasserstoffperoxid-Gehalt im Bereich 10 bis 75 Gew.%, vorzugsweise 30 bis 55 Gew.-%, eingesetzt wird.

9. Verfahren nach einem der Ansprüche 1 bis 8, **dadurch gekennzeichnet, dass** die Base gewählt ist aus der Gruppe Alkalihydroxid, Erdalkalihydroxid, Alkali- oder Erdalkalisalz einer gerad- oder verzweigtkettigen organischen Säure mit 1 bis 6 Kohlenstoffatomen.

10. Verfahren nach einem der Ansprüche 1 bis 9, **dadurch gekennzeichnet, dass** die Base gewählt ist aus der Gruppe Natriumhydroxid, Kaliumhydroxid, Alkaliacetat, Alkalipropionat.

11. Reaktionsgemisch, welches gemäß einem der Ansprüche 1 bis 10 erhältlich ist.

12. Verfahren zur Herstellung von 11(12)-Pentadecen-15-olid aus 1-Hydroperoxy-16-oxabicyclo[10.4.0]hexadecan (DDP-OOH), **dadurch gekennzeichnet, dass** eine Reaktionsgemisch nach Anspruch 11 eingesetzt wird.

13. Verfahren zur Herstellung von 11(12)-Pentadecen-15-olid aus 1-Hydroperoxy-16-oxabicyclo[10.4.0]hexadecan (DDP-OOH), **dadurch gekennzeichnet, dass** ein Reaktionsgemisch, das nach einem Verfahren gemäß einem der Ansprüche 1 bis 10 erhalten wurde, ohne Isolierung des DDP-OOH eingesetzt wird.

14. Verfahren nach Anspruch 12 oder 13, **dadurch gekennzeichnet, dass** die Umsetzung von DDP-OOH zu 11(12)-Pentadecen-15-olid in Gegenwart einer Kupferverbindung und eines hochsiedenden Verdünnungsmittels erfolgt, wobei die Kupferverbindung bei 20°C in dem hochsiedenden Verdünnungsmittel eine Löslichkeit von mindestens 0,5g / kg hochsiedendem Verdünnungsmittel aufweist.

15. Verfahren nach Anspruch 14, **dadurch gekennzeichnet, dass** das hochsiedende Verdünnungsmittel mindestens einen Siedepunkt von 170°C bei 5 mbar aufweist.

16. Verfahren nach Anspruch 14 oder 15, **dadurch gekennzeichnet, dass** das hochsiedende Verdünnungsmittel gewählt ist aus einer oder mehreren Verbindungen der Formel:
HX-[A]-YH
worin
X und Yunabhängig voneinander O oder N-R bedeuten, wobei R = H oder ein organischer Rest mit 1 bis 10 Kohlenstoffatomen ist, und
A ein organischer Rest mit bis zu 100 Kohlenstoffatomen ist.

17. Verwendung des Reaktionsgemisches nach Anspruch 11 zur Herstellung von 11(12)-Pentadecen-15-olid.

## Claims

1. Process for the preparation of 1-hydroperoxy-16-oxabicyclo[10.4.0]hexadecane (DDP-OOH), wherein 13-oxabicyclo[10.4.0]hexadec-1(12)-ene (DDP) and hydrogen peroxide are reacted in a diluent in the presence of a strong acid, the diluent has a pKa value of greater than or equal to 4.5 and the strong acid has a pKa value of less than or equal to 1.5, **characterised in that**, when the reaction has taken place, the strong acid is neutralised with at least 0.9 molar equivalent of a base.

2. Process according to claim 1, **characterised in that** the diluent has a pKa value in the range from greater than or equal to 4.5 to less than or equal to 25 and is preferably an organic acid.

3. Process according to claim 1, **characterised in that** the diluent is a straight- or branched-chained organic acid having from 2 to 6 carbon atoms or a mixture of organic acids having from 2 to 6 carbon atoms.

4. Process according to claim 1, **characterised in that** the diluent is acetic acid, propionic acid or a mixture of acetic acid and propionic acid.

5. Process according to any one of claims 1 to 4, **characterised in that** the strong acid has a pKa value of less than or equal to 1.0.

6. Process according to any one of claims 1 to 5, **characterised in that** the strong acid is a mineral acid or a straight- or branched-chained fluorinated carboxylic acid, preferably a perfluorinated carboxylic acid.

7. Process according to any one of claims 1 to 6, **characterised in that** the strong acid is selected from the group sulfuric acid, trifluoroacetic acid, perfluoropropionic acid, perfluorobutanoic acid, perfluoropentanoic acid, perfluorohexanoic acid.

8. Process according to any one of claims 1 to 7, **characterised in that** an aqueous hydrogen peroxide solution having a hydrogen peroxide content in the range from 10 to 75 wt.%, preferably from 30 to 55 wt.%, is used.

9. Process according to any one of claims 1 to 8, **characterised in that** the base is selected from the group alkali hydroxide, alkaline earth hydroxide, alkali or alkaline earth salt of a straight- or branched-chained organic acid having from 1 to 6 carbon atoms.

10. Process according to any one of claims 1 to 9, **characterised in that** the base is selected from the group sodium hydroxide, potassium hydroxide, alkali acetate, alkali propionate.

11. Reaction mixture obtainable according to any one of claims 1 to 10.

12. Process for the preparation of 11(12)-pentadecen-15-olide from 1-hydroperoxy-16-oxabicyclo[10.4.0]hexadecane (DDP-OOH), **characterised in that** a reaction mixture according to claim 11 is used.

13. Process for the preparation of 11(12)-pentadecen-15-olide from 1-hydroperoxy-16-oxabicyclo[10.4.0]hexadecane (DDP-OOH), **characterised in that** a reaction mixture obtained by a process according to any one of claims 1 to 10 is used without isolation of the DDP-OOH.

14. Process according to claim 12 or 13, **characterised in that** the reaction of DDP-OOH to 11(12)-pentadecen-15-olide is carried out in the presence of a copper compound and of a high-boiling diluent, wherein the copper compound has a solubility at 20°C in the high-boiling diluent of at least 0.5 g/kg of high-boiling diluent.

15. Process according to claim 14, **characterised in that** the high-boiling diluent has a boiling point of at least 170°C at 5 mbar.

16. Process according to claim 14 or 15, **characterised in that** the high-boiling diluent is selected from one or more compounds of the formula
HX-[A]-YH
wherein
X and Y independently of one another denote O or N-R, wherein R = H or is an organic radical having from 1 to 10 carbon atoms, and
A is an organic radical having up to 100 carbon atoms.

17. Use of the reaction mixture according to claim 11 in the preparation of 11(12)-pentadecen-15-olide.

## Revendications

1. Procédé pour la préparation de 1-hydroperoxy-16-oxabicyclo-[10.4.0]hexadécane (DDP-OOH), dans lequel du 13-oxabicyclo[10.4.0]hexadec-1(12)-ène (DDP) et du peroxyde d'hydrogène réagissent dans un diluant en présence d'un acide fort, le diluant présente une valeur pKa supérieue ou égale à 4,5 et l'acide fort une valeur pKa inférieure ou égale à 1,5, **caractérisé en ce qu'**après la réaction réalisée, on neutralise l'acide fort avec au moins 0,9 équivalent molaire d'une base.

2. Procédé selon la revendication 1, **caractérisé en ce que** le diluant présente une valeur pKa dans le domaine supérieur ou égal à 4,5 et inférieur ou égal à 25 et est de préférence un acide organique.

3. Procédé selon la revendication 1, **caractérisé en ce que** le diluant est un acide organique linéaire ou ramifié avec de 2 à 6 atomes de carbone ou un mélange d'acides organiques avec de 2 à 6 atomes de carbone.

4. Procédé selon la revendication 1, **caractérisé en ce que** le diluant est l'acide acétique, l'acide propionique ou un mélange d'acide acétique et d'acide propionique.

5. Procédé selon l'une quelconque des revendications 1 à 4, **caractérisé en ce que** l'acide fort présente une valeur pKa inférieure ou égale à 1,0.

6. Procédé selon l'une quelconque des revendications 1 à 5, **caractérisé en ce que** l'acide fort est un acide minéral ou un acide carboxylique fluoré linéaire ou ramifié, de préférence un acide carboxylique perfluoré.

7. Procédé selon l'une quelconque des revendications 1 à 6, **caractérisé en ce que** l'on choisit l'acide fort parmi l'acide sulfurique, l'acide trifluoroacétique, l'acide perfluoropropionique, l'acide perfluorobutyrique, l'acide perfluorovalérique, l'acide perfluorocaproïque.

8. Procédé selon l'une quelconque des revendications 1 à 7, **caractérisé en ce que** l'on utilise une solution aqueuse de peroxyde d'hydrogène avec une teneur en peroxyde d'hydrogène dans le domaine de 10 à 75 % en poids, de préférence de 30 à 55 % en poids.

9. Procédé selon l'une quelconque des revendications 1 à 8, **caractérisé en ce que** l'on choisit la base parmi un hydroxyde alcalin, un hydroxyde alcalino-terreux, un sel alcalin ou alcalino-terreux d'un acide organique linéaire ou ramifié avec de 1 à 6 atomes de carbone.

10. Procédé selon l'une quelconque des revendications 1 à 9, **caractérisé en ce que** l'on choisit la base parmi l'hydroxyde de sodium, l'hydroxyde de potassium, un acétate alcalin, un propionate alcalin.

11. Mélange réactionnel, lequel est obtenu selon l'une quelconque des revendications 1 à 10.

12. Procédé pour la préparation de 11(12)-pentadécèn-15-olide à partir de 1-hydroperoxy-16-oxabicyclo[10.4.0]hexadécane (DDP-OOH), **caractérisé en ce que** l'on utilise un mélange réactionnel selon la revendication 11.

13. Procédé pour la prépartion de 11(12)-pentadécèn-15-olide à partir de 1-hydroperoxy-16-oxabicylo[10.4.0]hexadécane (DDP-OOH), **caractérisé en ce que** l'on utilise un mélange réactionnel qui a été obtenu selon un procédé selon l'une des revendications 1 à 10 sans isoler le DDP-OOH.

14. Procédé selon la revendication 12 ou 13, **caractérisé en ce que** l'on réalise la transformation de DDP-OOH en 11(12)-pentadécèn-15-olide en présence d'un composé du cuivre et d'un diluant de point d'ébullition élevé, le composé du cuivre présentant à 20°C dans le diluant de point d'ébullition élevé une solubilité d'au moins 0,5 g/kg de diluant de point d'ébullition élevé.

15. Procédé selon la revendication 14, **caractérisé en ce que** le diluant de point d'ébullition élevé présente au moins un point d'ébullition de 170°C à 5 mbar.

16. Procédé selon la revendication 14 ou 15, **caractérisé en ce que** l'on choisit le diluant de point d'ébullition élevé parmi un ou plusieurs composés de la formule :
HX-[A]-YH
dans laquelle
X et Y représentent indépendamment 0 ou N-R, où R = H ou un reste organique avec de 1 à 10 atomes de carbone, et
A est un reste organique avec jusqu'à 100 atomes de carbone.

17. Utilisation du mélange réactionnel selon la revendication 11 pour la prépartion de 11(12)-pentadécèn-15-olide.
